# EUROPEAN PATENT SPECIFICATION

(11) **EP 3 828 282 B1**
(45) Date of publication and mention of the grant of the patent: **17.05.2023**
(21) Application number: 20851403.4
(22) Date of filing: 24.04.2020
(51) Int. Cl.: C12Q 1/68, G01N 21/15

(54) **SYSTEM WITH PROBE-STYLE ON-LINE BIOMASS MEASUREMENT APPARATUS ABLE TO UNDERGO HIGH TEMPERATURE STERILIZATION**
SYSTEM MIT FÜR HOCHTEMPERATURSTERILISATION GEEIGNETEM SONDENARTIGEM ONLINE-BIOMASSEDETEKTOR
SYSTÈME AVEC APPAREIL DE MESURE DE BIOMASSE EN LIGNE DE TYPE SONDE APTE À SUBIR UNE STÉRILISATION HAUTE TEMPÉRATURE

(30) Priority: 12.03.2020 CN 202010171350
(43) Date of publication of application: 02.06.2021
(73) Proprietor: Guangzhou Institute of Advanced Technology, Chinese Academy of Sciences, Guangzhou, Guangdong 510000 (CN)
(72) Inventor: WANG, Zhong, Guangzhou Guangdong 510000 (CN); WAN, Yuming, Guangzhou Guangdong 510000 (CN); DU, Xiaoying, Guangzhou Guangdong 510000 (CN); EICHSTAEDT, Olaf, Guangzhou Guangdong 510000 (CN); CUI, Jinming, Guangzhou Guangdong 510000 (CN)
(74) Representative: Zaboliene, Reda
(86) International application number: PCT/CN2020/086707
(87) International publication number: WO 2021/068482

(56) References cited:
- WO-A2-2008/157584
- CN-A- 101 368 207
- CN-A- 101 368 207
- CN-A- 101 750 280
- CN-A- 102 410 850
- CN-A- 107 148 937
- CN-A- 107 148 937
- CN-A- 109 520 968
- CN-U- 202 886 279
- US-A- 4 659 218
- US-A- 4 893 935
- US-A- 5 740 291
- US-A1- 2009 093 375
- US-A1- 2012 182 546

## Description

### Technical Field

The disclosure relates to the technical field of biological laboratory equipment, in particular to a probe-type online biomass detection apparatus.

### Background

In a microculture process, it is usually necessary to monitor growth statuses of microorganisms through methods such as Optical Density (OD) measurement. At present, the most common OD measurement method in laboratories is to manually take out a part of samples and use an OD detector to perform offline detection. Frequent manual sampling is cumbersome in operation, and a risk of infection with miscellaneous microbes is greatly increased in a sampling process.

There are also some online biomass detection apparatuses on the market that do not require manual sampling detection, which frees scientific researchers from the cumbersome manual operation, and also greatly reduces the risk of infection with miscellaneous microbes in the manual operation. At present, the online biomass detection apparatuses on the market may be divided into a contact type and a non-contact type according to whether they are in direct contact with cytosol or not.

Since the non-contact type online biomass detection apparatus is not in direct contact with the measured cytosol, there is no need to consider the sterilization; however, during cell culture, a fermentation tank for culturing cells often contains a large number of bubbles, which will cause a great interference to measurement values. Thus, how to avoid influence of the bubbles is the first problem that needs to be solved.

To the contact type online biomass detection apparatus, since a probe is in direct contact with the measured cytosol, the probe is required to be subjected to high-temperature sterilization together with the fermentation tank before use. Since the probe often includes a light source, a light receiving sensor, or other electronic components, how to ensure that the electronic components can resist a high temperature in an autoclave is a problem that needs to be addressed; on the other hand, the large number of bubbles in the fermentation tank will also cause the great interference to the measurement values, thus, how to avoid the influence of the bubbles is also the first problem that needs to be solved.

To the interference problem of the bubbles in the fermentation tank during measurement, in use of some online biomass detection apparatuses at present, on the one hand, measurement signals are filtered and optimized by software algorithms to a certain extent, and on the other hand, users are recommended to reduce the number of bubbles in ways such as reducing aeration rate and reducing agitation speed of the fermentation tank, but changing the aeration rate and agitation speed will often have a great influence on the whole fermentation process, so that use scenes are greatly restricted.

In bioengineering or fermentation engineering, a certain number of microorganisms are often inoculated into a liquid culture medium and then stored in a specific environment for culture. In order to monitor the growth statuses of the microorganisms in the culture medium, it is usually necessary to perform offline detection by methods such as OD measurement.

At present, the most common OD measurement method in laboratories is to manually take out a part of the samples by a scientific researcher, and use the OD detector to perform offline detection.

Patent CN105044038A discloses a non-invasive online biomass detection apparatus for shake-flaks culture, which includes a main control unit, a shake flask capable of containing a biological suspension, and a fixing casing covering the outside of the shake flask. The apparatus can realize online detection of a concentration of the biological suspension in the shake flask.

Patent CN101042327A discloses an optical fiber sensor system for online measurement of biomass concentration. The system includes an optical fiber sensor, a photoelectric conversion device, and a data processing display unit, and can realize measurement of the biomass concentration of different biological bacteria solutions.

At present, the most common offline OD measurement method in laboratories requires the scientific researcher to frequently perform manual sampling, which is cumbersome in operation and easy to cause infection with miscellaneous microbes.

The non-invasive online biomass detection apparatus for shake-flask culture, disclosed in the patent CN105044038A, is not in direct contact with the measured cytosol, and accordingly does not need to be sterilized. However, due to design limitations, the apparatus is only suitable for shake-flask measurement, and a shading treatment to the shake flask is also required.

The optical fiber sensor system for online measurement of biomass concentration, disclosed in the patent CN101042327A, can realize measurement of the biomass concentration of different biological bacterial solutions. The system does not specify whether it can be sterilized at a high temperature. The system adopts a principle of optical transmission measurement; the higher the cytosol concentration, the weaker the light signal received by the light receiving sensor, which directly leads to a relatively low measurement range (a general measurement range is that an OD600 value is about 0-4). It can be seen from the structure of the system that it is difficult for the system to avoid the influence of the bubbles in the cytosol.

Patent US 2012/182546 A1 discloses (see Figs. 2 and 14a- 14f, parr. [0087], [0105]-[0106]) a method and a device for simultaneous measurements of a sample in a multiphase system, and specifically, the fiber-optic probe may be a high temperature probe capable of supporting temperatures in the range of 1000°C, for use in industrial fluidized beds. The spectroscopic device comprises: a FT-IR spectrometer, a fiber-optic probe including an emitting fiber-optic, a receiving fiber-optic, a planar gold-coated mirror and the like.

Patent US 4893935 A discloses (see col. 2, I. 30-43, 58-61; col. 6, I. 3-7 and Figs. 2-3) an apparatus and a method for optical density measurements of biomass processes, and specifically discloses a probe, a light source, a detector, fiber optics and sample gap, and the like.

Patent US 4659218 A discloses a multi-probe system for measuring bubble characteristics gas hold-up, liquid hold-up and solid hold-up in a three-phase, and specifically discloses an apparatus for detecting bubble characteristics in a three phase system, a probe, gas bubbles, a beam splitter, a photodetector, and the like.

Patent US 5740291 A discloses a fiber optic sensor for sensing particle movement in a catalytic reactor, and specifically discloses a catalytic reactor, two separate probes, two receptor fibre optics, and the like.

Patent WO 2008/157584 A2 discloses an apparatus and methods for automatic insertion, debubbling, cleaning and calibration of a spectral probe during dissolution testing, and specifically discloses a dissolution testing apparatus including a bubble removal apparatus, a spectral probe, a dissolution test vessel containing dissolution media, a stirring device or other USP-type device, an automated actuator, a probe communicating with a vibration source or generator, and the like.

Patent CN 107148937 A discloses a method and a device for preventing bubble interference when infrared probe is used for water level detection. The device includes an infrared water level probe main body infrared probe, a conical water level detection area; an anti-bubble cover; an anti-bubble material. The conical water level detection area is completely provided within the anti-bubble cover. An anti-bubble material is provided at the bottom of the anti-bubble cover, such that the bubble in the water can not enter inner of the anti-bubble cover for an interference.

### Summary

In view of this, in order to solve the above-mentioned problems in the related art, the disclosure provides a probe-type online biomass detection apparatus, in which an optical fiber probe can be configured for sterilizing at high-temperature while interference of bubbles in a fermentation tank on measurement results can be eliminated.

The disclosure solves the above problems by means of the following technical solutions.

The probe-type online biomass detection system includes a probe-type online biomass detection apparatus and a fermentation tank, wherein the apparatus includes an optical fiber probe, a light source and light receiving sensor module, and a signal processing module;
the light source and light receiving sensor module includes a light source, and a light receiving sensor; and
measuring light emitted from the light source is received and transmitted to a measurement area through the optical fiber probe, cells in the measurement area has a reflection action to the light, the reflected light returns to the optical fiber probe, and received by the light receiving sensor through the optical fiber probe, a light intensity signal received by the light receiving sensor directly reflects the cell concentration, and the signal processing module calculates the cell concentration according to the light intensity signal received by the light receiving sensor;
the optical fiber probe is provided in the fermentation tank, and an axis of the optical fiber probe is parallel to an axis of the fermentation tank;
the optical fiber probe comprises a light guide component, a probe holder, and a bubble filter casing;
the bubble filter casing comprises a fluid inlet, at least one fluid outlet, and a measuring cavity; the fluid inlet is arranged at the bottom surface of the bubble filter casing, and the at least one fluid outlet is arranged at a lateral side of the bubble filter casing.

In an embodiment, the light source and light receiving sensor module is detachably connected to the optical fiber probe.

In an embodiment, the light guide component is arranged on the probe holder , and the bubble filter casing covers the light guide component, and is connected to the probe holder.

In an embodiment, the light guide component is a high-temperature resistant optical fiber.

In an embodiment, the bubble filter casing is cylindrical in shape, and the at least one fluid outlet on a cylindrical arc surface of the bubble filter casing is ensured to be not tangent to a cytosol flow direction during mounting.

In an embodiment, the bubble filter casing is cylindrical in shape, and the at least one fluid outlet on a cylindrical arc surface of the bubble filter casing is ensured to be perpendicular to a cytosol flow direction during mounting.

In an embodiment, the bubble filter casing is cylindrical in shape, a plurality of fluid outlets are arranged on circumference of a cylindrical arc surface of the bubble filter casing, so that at least one fluid outlet is ensured to be not tangent to a cytosol flow direction no matter which direction the bubble filter casing is mounted in.

In an embodiment, an end surface of the light guide component is mounted as or made into a bevel or an upward surface.

In an embodiment, an end surface of the light guide component is coated with a material that is not easy to be adhered with bubbles.

Compared with the related art, the disclosure at least has the following beneficial effects.
1) Online detection of biomass in a culture solution is achieved, and the cumbersome operation of manual sampling and the risk of infection with miscellaneous microbes are avoided.
2) The apparatus may be placed in an autoclave to be sterilized at high-temperature, thereby protecting the measured cytosol against being contaminated by the miscellaneous microbes.
3) The interference of the bubbles in the fermentation tank on the measurement results may be eliminated, thereby providing a guarantee to accuracy of the measurement results.

### Brief Description of the Drawings

In order to describe the technical solutions of embodiments of the disclosure more clearly, the drawings required to be used in the embodiments of the disclosure will be simply introduced below. It is apparent that the drawings described below are only some embodiments of the disclosure. Other drawings may further be obtained by those of ordinary skill in the art according to these drawings without creative efforts.
Fig. 1 is a schematic structural diagram of a probe-type online biomass detection apparatus according to Embodiment 1 of the disclosure;
Fig. 2 is a schematic structural diagram of a probe-type online biomass detection apparatus according to Embodiment 2 of the disclosure;
Fig. 3 is a schematic structural diagram of an optical fiber probe in Embodiment 3 of the disclosure;
Fig. 4(a) and Fig. 4(b) are schematic structural diagrams of a bubble filter casing in Embodiment 4 of the disclosure, where Fig. 4(a) is an axonometric drawing, and Fig. 4(b) is a sectional view;
Fig. 5(a) and Fig. 5(b) are schematic diagrams of a mounting orientation of an optical fiber probe in a fermentation tank in Embodiment 5 of the disclosure, where Fig. 5(a) is a front view, and Fig. 5(b) is a top view; and
Fig. 6 is a schematic structural diagram of an end surface of an optical fiber that is mounted at an angle of α with a horizontal plane in Embodiment 7 of the disclosure.

Description of Reference Signs:
1, optical fiber; 2, light source and light receiving sensor module; 3, signal processing module; 101, high-temperature resistant optical fiber; 102, probe holder; 103, bubble filter casing; 201, light source; 202, light receiving sensor; 1031, fluid inlet; 1032, fluid outlet; 1033, measuring cavity; and 00, fermentation tank.

### Detailed Description of the Embodiments

In order to make the above purposes, technical solutions and advantages of the disclosure more apparent and understandable, the following describes the technical solutions in the embodiments of the disclosure in detail with reference to the accompanying drawings and the specific embodiments. It is to be noted the described embodiments are a part rather than all of the embodiments of the disclosure. All other embodiments obtained by those of ordinary skill in the art based on the embodiments of the disclosure without creative efforts shall fall within the protection scope of the disclosure.

### Embodiment 1

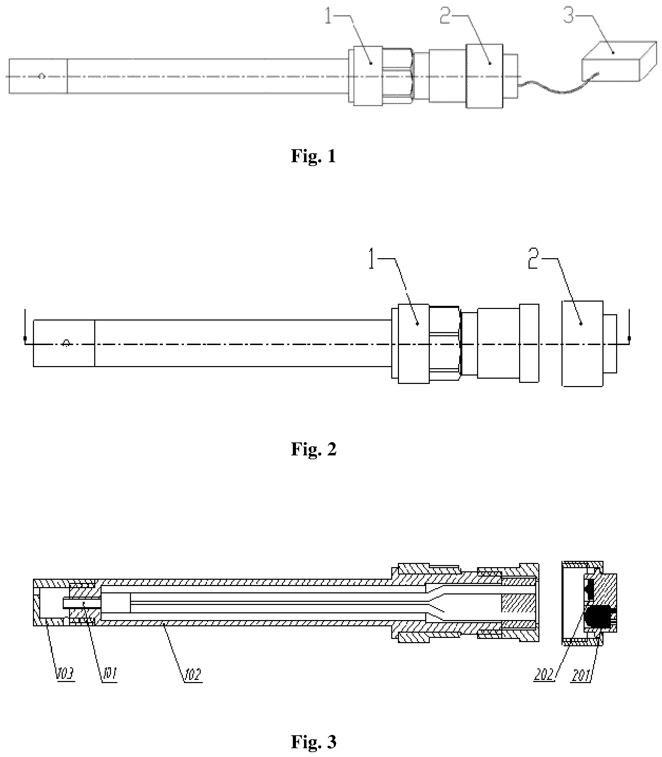

As shown in Fig. 1, the disclosure provides a probe-type online biomass detection apparatus, mainly including an optical fiber probe 1, a light source and light receiving sensor module 2, and a signal processing module 3.

The light source and light receiving sensor module 2 includes a light source 201, and a light receiving sensor 202.

The apparatus adopts a principle of reflected light measurement: measuring light is emitted from the light source 201 to a measurement area through the optical fiber probe 1; Cells in the measurement area has a reflection action to the light; the reflected light returns to the optical fiber probe 1, and reaches the light receiving sensor 202 through the optical fiber probe 1; a light intensity signal received by the light receiving sensor 202 may directly reflect the cell concentration, and the signal processing module 3 calculates the cell concentration according to the light intensity signal received by the light receiving sensor 202.

### Embodiment 2

As shown in Fig. 2, according to this embodiment based on Embodiment 1, in order to meet the requirements for high-temperature sterilization, a light source and light receiving sensor module 2 and an optical fiber probe 1 adopt a detachable design; when the optical fiber probe 1 is sterilized at high-temperature, the light source and light receiving sensor module 2 is detached, and is mounted again for detection after the sterilization is finished.

### Embodiment 3

As shown in Fig. 3, according to this embodiment based on Embodiment 1, in order to avoid interference of bubbles, a bubble filtering structure is designed in a measurement area of the probe, to reduce flow rate and number of bubbles in the measurement area. The optical fiber probe 1 includes a high-temperature resistant optical fiber 101, a probe holder 102, and a bubble filter casing 103; the high-temperature resistant optical fiber 101 is arranged on the probe holder 102, and the bubble filter casing 103 covers the high-temperature resistant optical fiber 101 and is connected to the probe holder 102.

### Embodiment 4

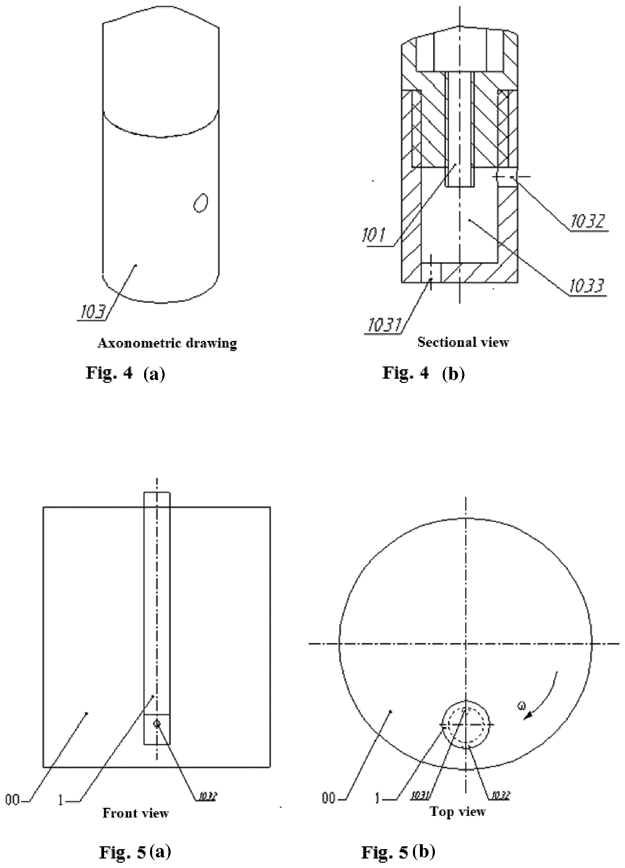

As shown in Fig. 4(a) and Fig. 4(b), according to this embodiment based on Embodiment 3, the bubble filter casing 103 includes a fluid inlet 1031, a fluid outlet 1032, and a measuring cavity 1033; the fluid inlet 1031 is arranged at the bottom surface of the bubble filter casing 103, and the fluid outlet 1032 is arranged at the lateral side of the bubble filter casing 103.

### Embodiment 5

As shown in Fig. 5(a) and Fig. 5(b), according to this embodiment based on Embodiment 4, a fluid outlet 1032 on a cylindrical arc surface of a bubble filter casing 103 is ensured to be not tangent to a cytosol flow direction during mounting (the best is a vertical direction as shown in Fig. 5(a) and Fig. 5(b)). A working principle includes: when the cytosol flows through the cylindrical arc surface, the flow rate of the cytosol is greater than the flow rate of the cytosol flowing through a cylindrical bottom plane; according to knowledge of fluid mechanics, the faster the fluid flow rate, the lower the pressure, an additional pressure difference is generated between the inlet at the cylindrical bottom plane and the outlet at the cylindrical arc surface due to flowing of the cytosol, so that the cytosol outside a measuring cavity 1033 continuously flows in from the inlet and flows out from the outlet, content of bubbles in the cytosol in the measuring cavity 1033 is relatively low and the flow rate is relatively low, which facilitates reducing interference of the bubbles on measurement results. Schematic diagrams of a mounting orientation of an optical fiber probe 1 in a fermentation tank 00 are shown as Fig. 5(a) and Fig. 5(b).

### Embodiment 6

According to this embodiment based on Embodiment 4, a plurality of fluid outlets 1032 are arranged on circumference of a cylindrical arc surface of a bubble filter casing 103, so that at least one fluid outlet is ensured to be not tangent to a cytosol flow direction no matter which direction the bubble filter casing is mounted in, and in this way, orientations of the fluid outlets 1032 are not strictly limited.

### Embodiment 7

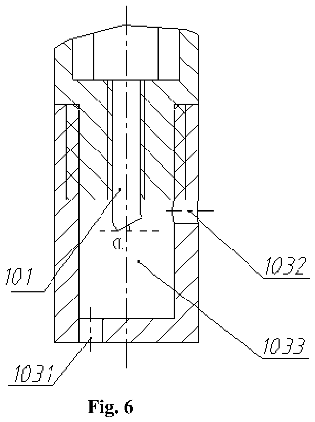

As shown in Fig. 6, according to this embodiment based on Embodiment 4, since bubbles in a measuring cavity 1033 are easy to accumulate on an end surface of an optical fiber to affect a measurement signal, in order to prevent the bubbles accumulating on the end surface of the optical fiber, the end surface of the optical fiber may be mounted as or made into a bevel or an upward surface, and may also be coated with a material that is not easy to be adhered with bubbles.

In the probe-type online biomass detection apparatus, the electronic components such as the light source, and the light receiving sensor are arranged by using a modular design, and a portion, which is required to touch the cytosol, of the optical fiber probe adopts a detachable design scheme, so that requirements for high-temperature sterilization are met. In the meantime, with regard to the influence of the bubbles in the fermentation tank on the measurement values, a bubble filtering structure is designed, to reduce flow rate and number of the bubbles in the measurement area; and the measurement signal is filtered and optimized through a software algorithm, so that the interference of the bubbles is eliminated finally.

Compared with the related art, the disclosure at least has the following beneficial effects.
1) Online detection of biomass in a culture solution is achieved, and the cumbersome operation of manual sampling and the risk of infection with miscellaneous microbes are avoided.
2) The apparatus may be placed in an autoclave to be sterilized at high-temperature, thereby protecting the measured cytosol against being contaminated by the miscellaneous microbes.
3) The interference of the bubbles in the fermentation tank on the measurement results can be eliminated, thereby providing a guarantee to accuracy of the measurement results.

The above embodiments only express several implementation modes of the disclosure, while the description is more specific and detailed, it should not be interpreted as a limitation to the patent scope of the disclosure. It is to be pointed out that those of ordinary skill in the art may make several modifications and improvements without departing from the concept of the disclosure, and these all fall within the protection scope of the disclosure. Therefore, the protection scope of the disclosure should be subject to the appended claims.

## Claims

1. A probe-type online biomass detection system, comprising a probe-type online biomass detection apparatus and a fermentation tank (00), an optical fiber probe (1), a light source and light receiving sensor module (2), and a signal processing module (3);
the light source and light receiving sensor module (2) comprises a light source (201), and a light receiving sensor (202); and
measuring light emitted from the light source (201) is received and transmitted to a measurement area through the optical fiber probe (1), cells in the measurement area has a reflection action to the light, the reflected light returns to the optical fiber probe (1), and received by the light receiving sensor (202) through the optical fiber probe (1), a light intensity signal received by the light receiving sensor (202) directly reflects the cell concentration, and the signal processing module (3) calculates the cell concentration according to the light intensity signal received by the light receiving sensor (202);
the optical fiber probe (1) is provided in the fermentation tank (00);
**characterised in that**
an axis of the optical fiber probe (1) is parallel to an axis of the fermentation tank (00);
the optical fiber probe (1) comprises a light guide component, a probe holder (102), and a bubble filter casing (103);
the bubble filter casing (103) comprises a fluid inlet (1031), at least one fluid outlet (1032), and a measuring cavity (1033); the fluid inlet (1031) is arranged at the bottom surface of the bubble filter casing (103), and the at least one fluid outlet (1032) is arranged at a lateral side of the bubble filter casing (103).

2. The probe-type online biomass detection system according to claim 1, **characterized in that**: the light source and light receiving sensor module (2) is detachably connected to the optical fiber probe (1).

3. The probe-type online biomass detection system according to claim 1, **characterized in that**: the light guide component is arranged on the probe holder (102), and the bubble filter casing (103) covers the light guide component, and is connected to the probe holder (102).

4. The probe-type online biomass detection system according to claim 3, **characterized in that**: the light guide component is a high-temperature resistant optical fiber (101).

5. The probe-type online biomass detection system according to claim 1, **characterized in that**: the bubble filter casing (103) is cylindrical in shape, and the at least one fluid outlet (1032) on a cylindrical arc surface of the bubble filter casing (103) is ensured to be not tangent to a cytosol flow direction during mounting.

6. The probe-type online biomass detection system according to claim 1, **characterized in that**: the bubble filter casing (103) is cylindrical in shape, and the at least one fluid outlet (1032) on a cylindrical arc surface of the bubble filter casing (103) is ensured to be perpendicular to a cytosol flow direction during mounting.

7. The probe-type online biomass detection system according to claim 1, **characterized in that**: the bubble filter casing (103) is cylindrical in shape, a plurality of fluid outlets (1032) are arranged on circumference of a cylindrical arc surface of the bubble filter casing (103), so that at least one fluid outlet (1032) is ensured to be not tangent to a cytosol flow direction no matter which direction the bubble filter casing (103) is mounted in.

8. The probe-type online biomass detection system according to claim 3, **characterized in that**: an end surface of the light guide component is mounted as or made into a bevel or an upward surface.

9. The probe-type online biomass detection system according to claim 3, **characterized in that**: an end surface of the light guide component is coated with a material that is not easy to be adhered with bubbles.

## Patentansprüche

1. Sondenartiges Online-Biomassedetektionssystem, umfassend eine Sondentyp-Online-Biomassedetektionsvorrichtung und einen Fermentationstank (00), eine optische Fasersonde (1), ein Lichtquelle- und Lichtempfangssensormodul (2) und ein Signalverarbeitungsmodul (3);
das Lichtquelle- und Lichtempfangssensormodul (2) eine Lichtquelle (201) und einen Lichtempfangssensor (202) umfasst;
von der Lichtquelle (201) emittiertes Messlicht empfangen und durch die optische Fasersonde (1) zu einem Messbereich übertragen wird, Zellen in dem Messbereich eine Reflexionswirkung auf das Licht haben, das reflektierte Licht zur optischen Fasersonde (1) zurückkehrt, und von dem Lichtempfangssensor (202) durch die optischen Fasersonde (1) empfangen wird, ein von dem Lichtempfangssensor (202) empfangenes Lichtintensitätssignal direkt die Zellkonzentration widerspiegelt und das Signalverarbeitungsmodul (3) die Zellkonzentration gemäß dem von dem Lichtempfangssensor (202) empfangenen Lichtintensitätssignal berechnet;
die optische Fasersonde (1) im Fermentationstank (00) vorgesehen ist; **dadurch gekennzeichnet, dass**
eine Achse der optischen Fasersonde (1) parallel zu einer Achse des Fermentationstanks (00) ist;
die optische Fasersonde (1) eine Lichtleitkomponente, einen Sondenhalter (102) und ein Blasenfiltergehäuse (103) umfasst;
das Blasenfiltergehäuse (103) einen Fluideinlass (1031), mindestens einen Fluidauslass (1032) und einen Messhohlraum (1033) umfasst; der Fluideinlass (1031) an der Bodenfläche des Blasenfiltergehäuses (103) angeordnet ist und der mindestens eine Fluidauslass (1032) an einer lateralen Seite des Blasenfiltergehäuses (103) angeordnet ist.

2. Sondenartiges Online-Biomassedetektionssystem nach Anspruch 1, **dadurch gekennzeichnet, dass** das Lichtquellen- und Lichtempfangssensormodul (2) lösbar mit der optischen Fasersonde (1) verbunden ist.

3. Sondenartiges Online-Biomassedetektionssystem nach Anspruch 1, **dadurch gekennzeichnet, dass** die Lichtleiterkomponente auf dem Sondenhalter (102) angeordnet ist und das Blasenfiltergehäuse (103) die Lichtleiterkomponente bedeckt und mit der Sondenhalterung (102) verbunden ist.

4. Sondenartiges Online-Biomassedetektionssystem nach Anspruch 3, **dadurch gekennzeichnet, dass** die Lichtleiterkomponente eine hochtemperaturbeständige optische Faser (101) ist.

5. Sondenartiges Online-Biomassedetektionssystem nach Anspruch 1, **dadurch gekennzeichnet, dass** das Blasenfiltergehäuse (103) zylindrisch geformt ist und der mindestens eine Fluidauslass (1032) auf einer zylindrischen Bogenfläche des Blasenfiltergehäuses (103) während der Montage während der Montage so sichergestellt ist, dass er nicht tangential zu einer Cytosol-Strömungsrichtung ist.

6. Sondenartiges Online-Biomassedetektionssystem nach Anspruch 1, **dadurch gekennzeichnet, dass** das Blasenfiltergehäuse (103) zylindrisch geformt ist und der wenigstens eine Fluidauslass (1032) auf einer zylindrischen Bogenfläche des Blasenfiltergehäuses (103) während der Montage so sichergestellt ist, dass er senkrecht zu einer Cytosol-Strömungsrichtung ist.

7. Sondenartiges Online-Biomassedetektionssystem nach Anspruch 1, **dadurch gekennzeichnet, dass** das Blasenfiltergehäuse (103) zylindrisch geformt ist, mehrere Fluidauslässe (1032) am Umfang einer zylindrischen Bogenfläche des Blasenfiltergehäuses (103) angeordnet sind, so dass mindestens ein Fluidauslass (1032) sichergestellt ist, dass es nicht tangential zu einer Cytosol-Strömungsrichtung ist, egal in welcher Richtung das Blasenfiltergehäuse (103) montiert ist.

8. Sondenartiges Online-Biomassedetektionssystem nach Anspruch 3, **dadurch gekennzeichnet, dass** eine Endfläche der Lichtleiterkomponente als Abschrägung oder nach oben gerichtete Fläche montiert oder zu dieser gemacht ist.

9. Sondenartiges Online-Biomassedetektionssystem nach Anspruch 3, **dadurch gekennzeichnet, dass** eine Endfläche der Lichtleiterkomponente mit einem Material beschichtet ist, das nicht leicht mit Blasen zu haften ist.

## Revendications

1. Système à sonde de détection de biomasse en ligne, comprenant un appareil à sonde de détection de biomasse en ligne et une cuve de fermentation (00), une sonde à fibre optique (1), un module de source de lumière et de capteur de réception de lumière (2), et un module de traitement des signaux (3) ;
le module de source de lumière et de capteur de réception de lumière (2) comprend une source de lumière (201) et un capteur de réception de lumière (202) ; et
la lumière de mesure émise par la source de lumière (201) est reçue et transmise à une zone de mesure à travers la sonde à fibre optique (1), les cellules dans la zone de mesure ont une action de réflexion sur la lumière, la lumière réfléchie retourne à la sonde à fibre optique (1) et est reçue par le capteur de réception de lumière (202) à travers la sonde à fibre optique (1), un signal d'intensité de lumière reçu par le capteur de réception de lumière (202) reflète directement la concentration cellulaire, et le module de traitement des signaux (3) calcule la concentration cellulaire en fonction du signal d'intensité de lumière reçu par le capteur de réception de lumière (202) ;
la sonde à fibre optique (1) est placée dans la cuve de fermentation (00) ;
**caractérisé en ce que**
un axe de la sonde à fibre optique (1) est parallèle à un axe de la cuve de fermentation (00) ;
la sonde à fibre optique (1) comprend un composant de guidage de la lumière, un support de sonde (102) et un boîtier de filtre à bulle (103) ;
le boîtier de filtre à bulle (103) comprend une entrée de fluide (1031), au moins une sortie de fluide (1032) et une cavité de mesure (1033) ; l'entrée de fluide (1031) est disposée sur la surface inférieure du boîtier de filtre à bulle (103), et au moins une sortie de fluide (1032) est disposée sur un côté latéral du boîtier de filtre à bulle (103).

2. Système à sonde de détection de biomasse en ligne selon la revendication 1, **caractérisé en ce que**, le module de source de lumière et de capteur de réception de lumière (2) est relié de manière amovible à la sonde à fibre optique (1).

3. Système à sonde de détection de biomasse en ligne selon la revendication 1, **caractérisé en ce que**, le composant de guidage de la lumière est disposé sur le support de sonde (102), et le boîtier de filtre à bulle (103) recouvre le composant de guidage de la lumière, et est connecté au support de sonde (102).

4. Système à sonde de détection de biomasse en ligne selon la revendication 3, **caractérisé en ce que**, le composant de guidage de la lumière est une fibre optique résistante aux hautes températures (101).

5. Système à sonde de détection de biomasse en ligne selon la revendication 1, **caractérisé en ce que**, le boîtier de filtre à bulle (103) est de forme cylindrique, et l'au moins une sortie de fluide (1032) sur une surface d'arc cylindrique du boîtier de filtre à bulle (103) est assurée de ne pas être tangente à une direction d'écoulement du cytosol pendant le montage.

6. Système à sonde de détection de biomasse en ligne selon la revendication 1, **caractérisé en ce que**, le boîtier de filtre à bulle (103) est de forme cylindrique, et l'au moins une sortie de fluide (1032) sur une surface d'arc cylindrique du boîtier de filtre à bulle (103) est assurée d'être perpendiculaire à une direction d'écoulement du cytosol pendant le montage.

7. Système à sonde de détection de biomasse en ligne selon la revendication 1, **caractérisé en ce que**, le boîtier de filtre à bulle (103) est de forme cylindrique, une pluralité de sorties de fluide (1032) sont disposées sur la circonférence d'une surface d'arc cylindrique du boîtier de filtre à bulle (103), de sorte qu'au moins une sortie de fluide (1032) est assurée de ne pas être tangente à une direction d'écoulement du cytosol quelle que soit la direction dans laquelle le boîtier de filtre à bulle (103) est monté.

8. Système à sonde de détection de biomasse en ligne selon la revendication 3, **caractérisé en ce que**, une surface d'extrémité du composant de guidage de la lumière est montée en biseau ou en surface ascendante.

9. Système à sonde de détection de biomasse en ligne selon la revendication 3, **caractérisé en ce que**, une surface d'extrémité du composant de guide de lumière est revêtue d'un matériau qui n'est pas facile à faire adhérer aux bulles.
